# EUROPEAN PATENT APPLICATION

(11) **EP 4 186 376 A1**
(43) Date of publication of application: **31.05.2023**
(21) Application number: 20736753.3
(22) Date of filing: 12.06.2020
(51) Int. Cl.: A23L 13/00

(54) **EDIBLE AND STERILIZABLE POROUS 3D SCAFFOLD AND USES THEREOF**

(71) Applicant: Biotech Foods S.L., 20018 Donostia-San Sebastián (ES)
(72) Inventor: VILA, Mercedes, 20018 Donostia-San Sebastián (ES); CORTIZO, Diego, 20018 Donostia-San Sebastián (ES)
(74) Representative: Balder IP Law, S.L.
(86) International application number: PCT/ES2020/070386
(87) International publication number: WO 2021/250291

(57) **Abstract**

The present invention relates to an edible and sterilizable macroporous three-dimensional (3D) tissue engineering scaffolds comprising a network of cross-linked biocompatible polymer, preferably a natural polymer. Moreover, the scaffold of the invention further comprises additives and living cells which adhere and proliferate, colonizing the entire surface of the scaffold and giving rise to a raw material for the later formation of tissue with high nutritive content and/or cultured meat, that may be subsequently processed into food for animal or human consumption without requiring modification or removal of the cells form the scaffold. Method of using the scaffolds to make cultured meat and/or tissues for being processed as food comprising the scaffold, are also described herein.

## Description

### TECHNICAL FIELD

The invention relates to an edible and sterilizable macroporous three-dimensional (3D) tissue engineering scaffolds comprising, preferably, a natural polymer. Moreover, the scaffold of the invention further comprises additives and living cells which adhere and proliferate, colonizing the entire surface of the scaffold and giving rise to a raw material for the later formation of *in vitro* engineered tissues, also known in the art as cultured meat, cultivated meat, cell based meat, cellular meat and/or clean meat, that may be subsequently processed into food for animal or human consumption without requiring modification or removal of the cells from the scaffold. Method of using the scaffolds to make cultured meat and/or tissues for being processed as food including the scaffold are also described herein.

### STATE OF THE ART

Human population growth, which is expected to increase continuously (reaching 9,770 million by 2050 and 11,180 million at the turn of the century), will bring along greater waste production and the nutrient requirements of our species will continue to grow as we do. Meat figures prominently among protein foods. As divulged in the information provided by the Food and Agriculture Organization of the United Nations, the FAO (http://www.fao.org/ag/againfo/themes/es/meat/backgr_composition.html), meat is defined by the Codex Alimentarius as "all parts of an animal that are intended for, or have been judged as safe and suitable for, human consumption".

In the last 40 years, global meat production has grown significantly, due mainly to the development of countries. This growth has limited the production system, adversely affecting the environment in terms of natural resources (land and water), human health (pandemics) and animal well-being (animal suffering), due to which the consumption of cultured meat significantly contributes to feeding all the inhabitants of the planet by helping to create a more sustainable system. The FAO estimates that in the next 40 years the demand for animal protein will continue to grow and, in turn, the challenges for satisfying that demand. The current production model will not be able to satisfy this demand if not combined with new strategies such as cultured meat or meat produced by *in vitro* cultivation of muscle cells previously extracted from an animal.

Most of the research carried out on cultured meat can be considered variants or applications of tissue engineering, which is the discipline of biomedicine which, by combining cells, materials and engineering tools, attempts to design functional biological structures for repairing, replacing or regenerating damaged tissues. The progress made in this field is based mainly on the use of three-dimensional scaffolds/structures for tissue growth in a large variety of biomedical applications such as the regeneration of bone tissue, heart tissue, liver tissue, etc.

The international patent application WO1999031222A1 discloses the industrial production of meat by *in vitro* cultivation of animal muscle cells sown on traditional two-dimensional matrices, such as for example collagen. Moreover, the US patent US7270829B2 discloses a meat product obtained by means of non-human tissue engineering and a method for producing said meat product. Said meat product comprises muscle cells cultured *ex vivo* and is used for food consumption. Muscle cells can grow and become attached to a support structure and can be derived from any non-human cell. The meat product can also comprise other cells such as fat cells or cartilage cells, or both, which are cultured *ex vivo* together with muscle cells. This document is silent about the support for the growth of the cells.

The patent application US 2006/0121006 A1, related to the use of cultured animal cells for the production of nutritional or therapeutic products wherein said cells are derived from rare or endangered species. Edible products containing meat, suitable for human and non-human consumption, whether as food or as food supplements, are also included. The invention also relates to the use of large-scale culture methods for the proliferation of the cells prior to being added to said products. The invention encompasses the methods for manufacturing the products, the products themselves and the methods and uses thereof. Preference is shown for the culture of cells anchored to supports, particularly for supports in the form of microstructures (microspheres, fibres, etc.) of different materials, such as collagen, chitin, polystyrene, polyester or polypropylene, the choice of which will depend on the final use of the product and on whether the microstructure can remain in the final product or not.

Cell growth supports include microspheres and microsponges, called microcarriers, appropriate for use in a bioreactor for cell cultures, which can be used to form an edible engineered meat product are also described in the patent application US 2015/0079238 A1. Specifically, the described edible supports include: (i) porous microvalves that can be used solely for culturing cells (for example, smooth muscle cells); (ii) scaffolds with attached cells that can be included in the final meat product, without requiring modification or removal of the cells thereof. In a particular embodiment, the edible scaffolds may be formed by crosslinked pectin, such as thiopropionylamide (PTP) and polypeptides containing RGD sequences, such as thiolated cardosin A. Lastly, said patent application also describes different synthesis methods of such edible scaffolds for manufacturing meat in artificial state. It is interesting to highlight that these inventors, in the US application US 2013/0029008 A1, described different methods to elaborate edible engineered meat using cultured cells. Thus, the final meat product was formed from a plurality of partially fused layers and wherein each layer contains fused multicellular bodies (non-human myocytes).

Scaffolds based on natural polymers have demonstrated great potential for medical and pharmaceutical applications. The matrices formed by these biopolymers, some with the capabilities of hydrogel behavior, offer a series of advantages in terms of biocompatibility and biodegradability in comparison with the matrices formed from synthetic polymers. Thanks to their inherent properties, such as their great capacity to absorb water (swelling/hydration), biopolymers are capable of efficiently encapsulating and releasing a large variety of hydrophobic and hydrophilic therapeutic molecules in a controlled manner, including nucleic acids, proteins, antibodies, etc. (M. Efentakis, Adv. Polym. Tech. 2011, 30, 110-121). In biomedical applications, they have been used, for example, for cell encapsulation, a process that involves trapping cells in the polymer to protect them from the immune system (G. Orive et al., Nat. Med. 2003, 9, 104). For successful cell encapsulation, is important to maintain an appropriate diffusion balance for transporting oxygen and nutrients to the cells, increasing the porosity and permeability of the biopolymers.

The preparation of macroporous 3D scaffolds using different techniques based on natural biopolymers has been a strategy adopted for tissue regeneration (CY, Chen et al., Theranostics. 2015, 5, 643-655), although this work has been mainly carried out at small scale and in basic research. Normally, in this research it has been proposed that the polymeric matrices intended for tissue regeneration become slowly degraded as the new tissue is formed. As regards the food industry, on being formed mainly from polysaccharide units, they are key components in many formulations, their main applications being as emulsifying, gelling and/or stabilising agents (E. Bouyer et al., Int. J. Pharm. 2012, 436, 359-378).

Given the growing demand for substitutes for meat obtained directly from animals, it would be interesting to develop a matrix or scaffold which can be constituted by a natural polymer approved for food use, having a 3D structure and a large surface area, that may serve as a support for animal cells in the production of cultured meat, even in large-scale bioreactors, and which can remain in the final product due to being composed by natural biopolymer approved for food use.

### DESCRIPTION OF THE INVENTION

The present invention provides an edible and sterilizable macroporous three-dimensional (3D) tissue engineering scaffold, hereinafter, the scaffold of the invention, comprising at least a biocompatible polymer. The scaffold of the invention is composed mainly of biocompatible polymers of natural or synthetic origin whereto the cells, preferably mammal cells with the condition that they are non-human cells, may adequately adhere and proliferate, until colonizing the entire useful surface of the scaffold, wherein the cells are preferably, adherent cells.

One of the main challenges of supporting large scale cultured meat growth using the scaffolding technique is the compatibility of the scaffolds with the sterilization techniques used in large scale bioreactors. It is important to adapt the scaffolds to achieve a cost-efficient process for large volume productions of food products. These sterilizations process is based on a combination of steam, pressure and time. The process is operated at high temperature and pressure in order to kill microorganisms and spores. The scaffolds known in the art are not suitable to accomplish this process without losing its properties for tissue engineering.

On the contrary, the scaffold of the invention has the surprising advantage of being sterilizable preferably by hot steam since the biocompatible polymers selected for composition thereof are capable of resisting high water steam pressures at hot temperatures without degradation and/or denaturalization. It is important to note that the scaffold of the invention keeps intact its composition and structure after being subjected to a sterilization process, preferably by hot steam, allowing proper cell adhesion and tissue formation. Additionally, the sterilization process of the scaffold of the invention not only does not change the applicability of the materials due to possible degradation and/or denaturalization, but even improves the mechanical properties for supporting cell proliferation. Therefore, the scaffold of the invention could be sterilizable by any method known by the skilled in the art, preferably by hot steam without losing its properties as scaffold for cell proliferation, facilitating its incorporation on industrial processes requiring low cost and high-volume production.

Accordingly, the present invention discloses an edible and sterilizable, preferably by hot steam, macroporous 3D tissue scaffold wherein the macroporous are interconnected and wherein the scaffold comprises at least a biocompatible polymer, proposed as a support material for mammal cell growth, proliferation and differentiation, preferably, non-human cells, more preferably, adherent non-human cells, which may be used to obtain cultured meat.

As opposed in the known state of the art relating to cultured meat which proposes the idea of using the concept of tissue engineering for growing cultured meat but does not clearly define the adequate supports or is not materialized in an example of tissue engineering, the present invention proposes, for the first time, the use of an sterilizable edible 3D macroporous macrostructures comprising biocompatible polymer, preferably from natural origin, as scaffolds for the proliferation of cultured meat following the guidelines defined for tissue engineering, and adaptable to large scale manufacturing processes.

Thus, in a first aspect, the present invention refers to an edible and sterilizable, preferably by hot steam, macroporous 3D tissue engineering scaffold comprising a biocompatible polymer and interconnected macropores, preferably wherein the macropores and the scaffold comprises living cells.

As used herein, the term "edible" refers to materials, preferably food material intended for oral contact or consumption by eating.

The edible scaffold described herein are appropriate for the biofabrication of engineered tissues, also named or known for the skilled person as cultured meat, cultivated meat, cell based meat, cellular meat and/or clean meat, which are suitable for human consumption. For example, described herein are edible and sterilizable, preferably by hot steam, macroporous 3D tissue engineering scaffolds for use in forming a tissue with high nutritive content and/or a cultured meat product. Although the scaffolds and methods of synthesis and uses thereof described herein are primarily directed to comestible cultured meat products, such scaffold may also find application in cell therapy for animals, including mammals and humans, wherein it is desired to do cell expansion in the absence of animal-derived products.

As used herein, the terms "sterilizable" or "sterilize" refer to a procedure of destroying all microorganism, preferably pathogenic microorganism in or on given environment or material, in order to prevent the spread of infection. This is usually done by using heat, radiation, or chemical agents. In a preferred embodiment, the sterilization process is performed by hot steam.

As used herein, the terms "scaffold" or "support" used interchangeably thorough the present disclosure, and refer to the 3D matrix or material that allows the attachment and the proliferation of the cells, or other compounds such as additives, or likes, according to the present invention. "Attachment", "attach" or "attaches" as used herein, refers to cells that adhere directly or indirectly to the scaffold as well as to cells that adhere to other cells.

As used herein, the term "biocompatible polymer" refers to a synthetic or natural material/polymer that is, for example, non-toxic to biological systems and/or congruent with biological processes. In this respect, biocompatibility of polymer materials denotes minimal, negligible, or no risk of immuno-rejection, injury, damage and/or toxicity to living cells, tissues, organs, and/or biological systems.

In a preferred embodiment, the biocompatible polymer is a natural polymer. In an illustrative embodiment, the natural polymer, is anyone which can be able to resist a sterilization process at high pressures and temperature without degradation and/or denaturalization. Thus, the natural polymer or any derivative thereof is for example, but not limited to, polypeptides, polynucleotides, natural resins, rubbers, natural polyesters and polysaccharides. In a more preferred embodiment, the natural polymer is selected from the list consisting of: alginate, chitosan, starch, dextran, pullulan, heparin, heparin sulphate, cellulose, hemicellulose, glucomannan, agar, xanthan, guar gum, chondroitin sulphate, gelatine, chitin, a polysaccharide, a glycosaminoglycan, or any combinations thereof. In a more preferred embodiment, the natural polymer is selected from chitosan and/or alginate.

In the particular case of use gelatin as natural polymer, it is not sterilizable by hot steam and high pressure since it is partially hydrolyzed and denatured if submitted to these procedures. In the case that this natural polymer is inside the scaffold as small traces mixed with other biopolymer such as chitosan, the modification of scaffold properties is minimum still maintaining their adhesive properties.

These natural polymers can be directly used without any treatment from commercial sources or alternatively, chemical modifications can be carried out before being used to improve the properties of the scaffold of the present invention, in any of the steps of its production. As used herein, the term "derivative" is a similar compound obtained by chemically changing a part of a compound, but having the same or even better properties as the original compound. Thus, the derivatives of the natural polymers of the present invention refers to chemical modifications comprising the incorporation of different groups such as amino groups, aldehydes groups, carboxylic acids groups, amides groups, ketones groups, esters groups, alkoxy groups, sulphates groups, phosphates groups, or likes.

The scaffold of the invention comprises the natural polymer mentioned above, having the advantage of being composed of natural biopolymers approved for food use by the competent institutions such as, for example, the U.S. Food and Drug Administration (FDA) and the European Food Safety Authority (EFSA). In this sense, the edible and biocompatible polymers used in the scaffold of the invention are designed so that the scaffold be edible and does not have to be extracted from the final product, i.e. in order to avoid the collection process.

In the present invention is highly recommended to avoid the use of natural polymer selected from the list consisting of collagen, albumin (all types and forms), caseins, hyaluronic acid, fibrin, fibronectin and elastin, among others as they are not suitable for hot steam sterilization process.

In another preferred embodiment, the biocompatible polymer is a synthetic polymer or any variant thereof. Examples of synthetic polymers include, but not limited to, synthetic polypeptides from microorganisms or prepared by chemical synthesis techniques, bioesters, obtained from the extraction from microorganisms or prepared by chemical synthesis from their monomers such as polylactic acid, polyglycolic acid, poly(lactic-co-glycolic) acid, polyhydroxyalkanoates, or any other synthetic polymer prepared from generally recognized natural monomers such as monosaccharides (such as glucose, fructose, manose, galactose or any derivatives or variants such as glycosamines, aminosugars,... or likes), aminoacids or any derivative, nucleotides or any derivative, fatty acids or any derivative, or likes in any combination.

In a more preferred embodiment, the molecular weight (Mw) of the biocompatible polymers, or any derivatives thereof, range from 1000 Da to 5000000 Da, preferably from 10000 to 1000000 Da, more preferably from 100000 to 500000 Da.

The scaffold of the invention has macropores, preferably hierarchical and interconnected macropores, which have an average pore size is in the range of 1 µm to 10000 mm. In particular embodiments, the average pore size ranges from 50 to 1000 µm, preferably, from 50 µm to 100 µm, more preferably, from 100 µm to 1000 µm, or even more preferably, from 100 µm to 500 µm. In order to obtain larger macropores, a specific mould can be used in the method for the production of the scaffold of the invention, which makes it possible to obtain transverse or longitudinal pores along the scaffold having the average pore size mentioned above. In this sense, the arrangement of pores obtained through the formation of water crystals (solvent), together with the transverse macropores enables the obtainment of biopolymeric scaffolds with interconnected and hierarchical porous structures. The final pore size of the scaffold of the invention is influenced by the concentration of the polymer used in its formation. Furthermore, hydrogels with a high polymer concentration give rise to smaller pore sizes. In this case, the production process of the 3D macroporous scaffold gives pore size range between 50 µm to 10.000 µm, preferably 100 µm to 1000 µm, more preferably 100 and 500 µm.

In another preferred embodiment, the percentage by weight/volume of the biocompatible polymers mentioned herein or any combinations thereof, dissolved in an aqueous solution, organic solvents, cultured media, or in any combinations thereof in which is dissolved, ranges from 0.5% to 16%. In another preferred embodiment, the percentage by weight/volume ranges from 1% to 8 %, more preferably ranges from 1.5% to 4%, more preferably yet 1.5%, or alternatively in any of the possible relative ratios when there is more than one biocompatible polymer in the final scaffold.

In another preferred embodiment, the scaffold of the invention further comprises living cells, preferably non-human living cells, and more preferably adherent non-human living cells, which are homogeneously distributed though the scaffold. Many self-adhering cell types may be used to form the cultured meat products described herein. In some embodiments, the cultured meat products are designed to resemble traditional meat products and the cell types are chosen to approximate those found in traditional meat products. For the purposes of the present disclosure, cells used for culture along the scaffold of the invention to provide the cultured meat or tissues with high nutritive content, described herein, may include, without limitation, e.g., muscle cells, muscle progenitor cells, preferably skeletal muscle cells; smooth muscle cells, stromal cells, satellite cells, fibroblasts cells, myoblast cells, endothelial cells adipose cells such as adipocyte progenitor cells, hepatocytes, cardiomyocytes, etc.,. Thanks to its structure, the scaffold of the invention enables the proliferation and differentiation of various cell types such as, inter alia, myoblasts/myotubes/fibroblasts (muscle cells), endothelial cells, adipocytes (adipose cells), yielding cultured meat suitable for human consumption with the protein-fat content desired by the end consumer as the final product. As regards the cell types used for the production of cultured meat, it should be noted that the scaffold of the invention enables the proliferation and differentiation of a broad range of cell lines, such that we could perform different culture of a single cell type, for example, culture of myoblasts, which are differentiated to myotubes to form meat muscle tissue (protein). With these features, the final product is fat-free unless cell co-cultures of myoblasts with adipocytes are performed, a process that would also be possible if desired. In this manner we can influence the final features of the product with the objective of making it more attractive to the consumer and adapt it to the consumer's needs.

In a further preferred embodiment, the living cell lines cultures along the scaffold of the present invention could be obtained from various animal sources, such as, without limitation, mammals such as porcine, bovine, ovine, horse, dog, cat; avian; reptile; fish; amphibians; crustaceans or likes. This opens the doors to a huge market, since it offers a very diverse offering to end consumers.

In order to provide a scaffold with improved characteristics, it can be formed and/or combined with other molecules/substances/biomolecules selected from an additive, bioactive molecules, and/or a cross-linker agent, or any combination thereof, which having a high nutritional value or providing improved texture or adding flavor to the final scaffold. These compounds or substances may also enhance the adhesion properties and the proliferation of the future cells which can be seeded and cultured with the scaffold.

Thus, in another preferred embodiment, the scaffold of the present invention further comprises bioactive ligands. The bioactive ligands specifically interact with one or more biomolecules of cells or bind biomolecules that interact with biomolecules of the cells that are distributed within the scaffold or that proliferate within or migrate into the scaffold. Such interactions are effective to direct cell fate or induce the cells to form a tissue. These compounds or substances may also enhance the adhesion properties and the proliferation of the future cells which can be seeded and cultured with the scaffold. The identity of the bioactive ligands will depend upon the identity of the target cells, and some examples of suitable bioactive ligands include: carboxyl, amine, phenol, guanidine, thiol, indole, imidazole, hydroxyl, sulfate, norbornene, maleimide, laminin, fibrinogen, peptide sequences, adhesion molecules such as inmuglobulin-superfamiliy, cadherins (E-, N-, P-,...), selectins (P-, E-, L-,...) or integrins; fibronectins, poly-L-ornithine, collagen, vitronectins, lectin, poly-ornithine, poly-L-lysine, poly-D-lysine, cyclic peptides, RGD-containing peptides (Arg-Gly-Asp), RGDS-containing peptides (Arg-Gly-Asp-Ser), or any other compound or substance recognized by an expert in the field that promotes cell adhesion to the scaffold. Preferably, the bioactive molecules of the present disclosure refer to those substances that resist the sterilization process without affecting significantly their capacity to promote the cell adhesion. In a preferred embodiment, the bioactive molecule is poly-L-lysine, more preferably, poly-ε-lysine.

Poly-ε-lysine (ε-poly-L-lysine, EPL) is a naturally occurring antibacterial cationic peptide, a homopolymer of L-lysine, that is water soluble, edible, biodegradable, and non-toxic to humans and to the environment. Poly-ε-lysine is commonly used as a natural food preservative, emulsifying agent, as well as in cosmeceutical and pharmaceutical industry. In this sense, the Poly-ε-lysine is commonly used in in food applications such as boiled rice, cooked vegetables, soups, noodles, sliced fish (sushi) and meat.

This is the first time that the poly-ε-lysine is used as bioactive molecule, preferably as bioactive ligand for improving cell adhesion in *in vitro* cell cultures. As used in the present invention, the poly-ε-lysine is not only found on the surface of the scaffold of the invention, but also within it, being part of the overall 3D structure of the scaffold, thus allowing and improving cell adhesion thereon. Moreover, due to the natural antimicrobial activity of the poly-ε-lysine against yeast, fungi and bacteria, it helps to inhibit the growth of such pathogenic microorganisms in the scaffold of the invention.

In addition, poly-ε-lysine is already available in large quantities and at a low-price to the food industry, allowing the industrialized use thereof in the production of cultured meat. Thus, this property in conjunction with the surprising advantage of the scaffold of the invention characterized in that are sterilizable, preferably by hot steam, without degradation and/or denaturalization of the biocompatible polymers selected for composition thereof, make them suitable production in large scale industrial reactors, requiring, as previously mentioned, a very low production cost.

The additive including, but not limited to molecules/substances with high nutritional value or providing improved texture or adding flavor to the final product according to the present invention, and moreover are those substances that resist the sterilization process without affecting significantly their capacity to promote the cell adhesion. The additive are selected from the list consisting of: a flavoring, a flavor enhancer, a colorant, a color enhancer, salts, acidity regulators, thickeners, emulsifiers, stabiliser, a nutritional enhancer, such as vitamins, amino acids, fiber; fructo-oligosaccharides; inulins; beta carotene; omega fatty acids; spirulinas; probiotics; prebiotics; saponins; antioxidants; essential fatty acids; minerals; and likes, or any combinations thereof. The additives selected for the scaffold of the present invention are preferably from natural origin or source. Additionally, the term additive also encompasses compounds which enhance the adhesion properties and the proliferation of the future cells which can be seeded in the scaffold.

Examples of suitable cross-linkable groups are hydroxyl groups, carbonyl, groups, aldehyde groups, carboxylate group, carbonate group, carboxyl groups or derivatives, carboxamide groups, imine groups, imide groups, thiol groups and/or any other group, polyelectrolytes, inorganic ions or any combinations thereof.

In another preferred embodiment, the scaffold of the invention could be in the form of hydrogel. In a preferred embodiment, the scaffold of the invention in the form of hydrogel is useful for supporting the proliferation of the cells while forming the tissue, and additionally, can be uses as stabilizer, texturiser and/or a nutrient carrier, giving the cultured meat very high added value. Additionally, the scaffold of the invention in the form of hydrogel is useful as depots for targeted molecule delivery. Introducing a hydrogel scaffold core to culture meat provides a depot to encapsulate molecules for cellular proliferation and differentiation during the production process, as well as improve visual and nutritional content of the final tissue or cultured meat product.

In another preferred embodiment, the scaffold of the present invention is saturated with a cell culture medium. In some embodiments, the scaffolds are saturated with medium that facilitates cell proliferation or survival, i.e. a cell growth medium. In a more preferred embodiment, the cell growth medium is selected from commercially available classical media such as Dulbecco's Modified Eagle's Medium (DMEM, MEM) and its variants, or specifically defined culture mediums for each cell type used, known by the skill person in the art. In some embodiments, the scaffold may be impregnated with a biomolecule or other material intended for delivery to the food, preferably meat.

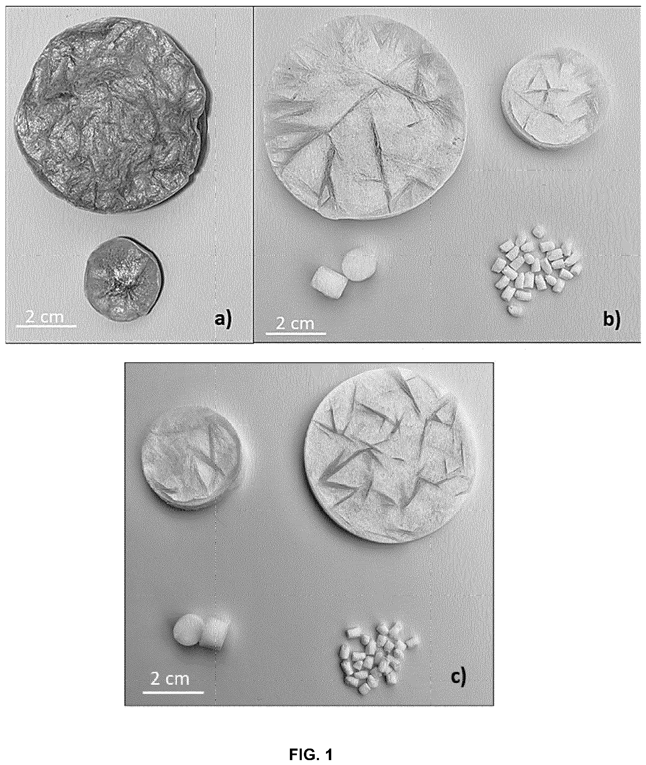

The structure of the scaffold of the present invention are shaped as desired. The shape of the scaffold of the present invention may be regular (e.g., cylindrical, spherical, rounded, rectangular, square etc.) or irregular. In addition to having the advantage of being edible and not having to be removed to prepare a final ready-to-eat meat food product, most of the sample polymers mentioned serve to give added value to products that would otherwise be completely discarded. For example, **Fig. 1** illustrate variations of scaffolds having cylindrical and disk-shapes. Any of these shapes may be porous.

As opposed to other previously proposed support structures for cell culture intended for obtaining cultured meat, the scaffold of the present invention is easily sterilizable inside large bioreactors, and its size makes it adequate for large-scale production and which has the advantage of being a prepared structure with edible materials. In this sense, the scaffold of the invention has the surprising advantage of being sterilizable since the biocompatible polymers selected for composition thereof are capable of resisting high water steam pressures at hot temperatures without degradation and/or denaturalization. It is important to note that the scaffold of the invention keeps its composition and structure after being subjected to a sterilization process, allowing proper cell adhesion and tissue formation. Additionally, the sterilization process of the scaffold of the invention not only does not change the applicability of the materials due to possible degradation and/or denaturalization, but even improve the mechanical properties for supporting cell proliferation. Therefore, the scaffold of the invention could be sterilizable by any method known by the skilled in the art, preferably by hot steam without losing its properties as scaffold for cell proliferation, facilitating its incorporation on industrial processes requiring low cost and high-volume production. In another preferred embodiment, the sterilization of the scaffold of the present disclosure is performed by a physical or chemical sterilization, preferably a physical sterilization process, which is selected from the list consisting of: hot steam, dry heat, tyndallisation, and ionizing or non-ionizing radiation, preferably hot steam. In another more preferred embodiment, the sterilization process is performed by hot steam in an autoclave or bioreactor at temperatures which ranges from 121°C (250°F) to 134°C (273°F), and at a pressure which ranges from 0.5 to 1.05 bar, for time which range from 10 to 60 min, more preferably, the sterilization by hot steam is performed for 20 min at a temperature for at least 121°C and at a pressure of 1,05 bar.

Moreover, thanks to the highly macroporous structure of the scaffold as it has been mentioned previously, it allows the transport of nutrients, thereby facilitating cell adhesion and proliferation, and giving rise to a raw material that can be subsequently processed in food prepared for consumption. Furthermore, the scaffold of the invention distributes the cells in the complete 3D space Until now, these scaffolds had never been envisaged for this purpose, since the large-scale cultured meat production technique is in its beginnings. The concept of these scaffold for producing cultured meat is novel and the application of the materials used to obtain the scaffold, in this form of highly porous edible and sterilizable scaffold, is also novel.

Therefore, the biocompatible polymers used in the scaffold of the present invention as mentioned above, and consequently, the scaffold of the invention comprising them, have the following advantages:
(i) it is also used as a binder, giving the growing meat better texture and compaction;
(ii) it improves the dispersal, bioavailability and absorption of nutrients in the digestive process stage;
(iii) taking into account that the biocompatible polymer could be a biopolymer with hydrogel properties, the scaffold where the cells grow can also act as a viscosifier and gelling agent, providing a texture pleasant to the taste and touch;
(iv) it can also act as a flavour and colour booster, since it acts as a release system *per* se similar to a drug-release system (carrier);
(v) it also acts as a selective delivery system for recognized health-beneficial components in food and/or free amino acids, thereby opening a wide range of possibilities for using these edible structures as possible carriers of vitamins, minerals, nucleic acids, or other biomolecules.

The scaffold of the invention satisfies fundamental requirements of tissue engineering, including: highly interconnected macropores that promote nutrient diffusion and facilitate cell proliferation, spreading, cell migration and cell-cell communication. Large internal surface area produces a large capacity for the proliferation of cells; distribute cells in 3D, facilitate nutrient transport, support cell migration and proliferation and facilitate mechanical support for the newly production of tissues.

The scaffolds of the present invention, as described above, are suitable for a variety of applications, including further regenerative medicine and tissue engineering. The scaffolds are particularly useful as tissue engineering. Thus, they may be used to facilitate cell proliferation and tissue formation. The macroporosity is suitable to allow cellular migration, formation of intracellular matrix, supply of nutrients, angiogenesis, and the like. Moreover, additional advantage of the scaffold of the present invention for use in regenerative medicine and tissue engineering is that is biocompatible.

The scaffolds of the invention are compatible with various types of reactors or bioreactors, even those wherein electric currents will be applied to stimulate cell proliferation, and they are also electrically conductive.

All the definitions and terms mentioned above for the first aspect of the present disclosure, apply mutatis mutandis to the further aspects of the present disclosure mentioned below.

In a further aspect, the present invention refers to a method for obtaining the scaffold of the present invention, wherein the method comprises:
a) preparing a solution of the biocompatible polymer according to the present invention and a suitable solvent, preferably a culture media,
b) Adding the solution of step a) into molds to give the desired shape and dimensions, and freeze, preferably at a temperature lower than the freezing temperature of the solution,
c) Lyophilizing the freeze-scaffold obtained in step b),
d) Curing the lyophilized scaffold of step c), and
e) Sterilization of the scaffold, preferably by hot steam.

In a preferred embodiment of the method for obtaining the scaffold, the selected biocompatible polymer, preferably a natural polymer as mentioned above, is dissolved at the required concentration, in a solvent selected from the list consisting of: aqueous solutions, organic solvents, culture media, or any combinations thereof, in step a).

In another preferred embodiment, the organic solvents are selected from the list consisting of: ethanol, dioxane, acetone, acetic acid, iso-propyl alcohol, acetonitrile, dimethylsulfoxide, trifluoracetic acid or any other, as pure or as a co-solvent in aqueous solutions in any appropriate ratio.

In another preferred embodiment, the culture media refers to a solution containing nutrients to support cell survival under conditions in which the selected cells can grow. Examples of suitable culture media are selected from commercially available classical media such as Dulbecco's Modified Eagle's Medium (DMEM, MEM) or any variants thereof. In this sense, the skilled person knows what the specific culture media is suitable for each cell type used.

In another preferred embodiment, the concentration of the biocompatible polymer of the invention, or any combinations thereof, ranges from 0.5% to 16% (w/w), preferably between 1% to 8%, more preferably between 1.5% to 4%, or alternatively in any of the possible relative ratios when there is more than one biocompatible polymer in the final scaffold.

In another preferred embodiment, the pH of the solution comprising the solvent and the biocompatible polymer can be neutral or can be a pH which can ranges from 1 to 14, preferably from 2 to 10, to enhance the solubility of the polymer depending on the polymer used. In order to improve the solubility of the selected biocompatible polymers, the temperature of step a) can be increased from room temperature until 180°C. In a preferred embodiment, the temperature ranges from 22°C to 70°C, more preferably from 30 to 70°C, more preferably yet, from 35°C to 65°C.

In another preferred embodiment, the scaffold of step b) is formed by molding the dissolution via extrusion directly.

In another preferred embodiment, the freezing temperature of step b) ranges from - 15°C to -80°C.

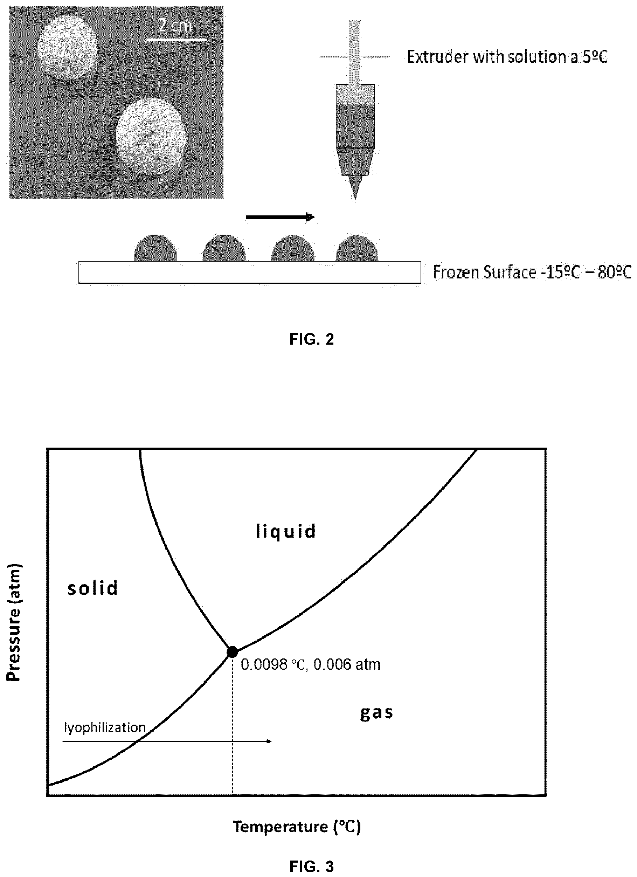

In another preferred embodiment, the lyophilization of step c) is performed for at least a period which ranges from 16h to 96h, preferably from 24 to 72h, more preferably from 18 to 24, at a condenser temperature which ranges from 25°C to -100°C, preferably from 25°C to -100°C,more preferably from 20°C to -50°C, and more preferably from - 15°C to -45°C, and to a pressure from 0.01 to 0.026 Pa (1 to 2.6 x 10⁻⁴ mbar), more preferably from (100 to 40 Pa (1 to 0.4 mbar), more preferably from 20 to 40 Pa (0.2 to 0.4 mbar). In order to help sublimation, lyophilized shelves may be heated up to 20 °C. A temperature difference of 15 °C to 20 °C between sample and condenser is recommended. In another preferred embodiment, the lyophilized scaffolds are then crosslinked with an adequate crosslinker and/or a surface coating treatment, wherein this treatment is carried out with substances or biomolecules that enhance the cell adhesion and proliferation. The parameters used in the process of lyophilization, such as time, temperature and pressure, are selected in a manner that the scaffolds loose at least 98% of the initial water. See **Fig. 3** wherein the phase diagram of water indicates the triple point when lyophilization occurs.

In another preferred embodiment, curing process of step d) is preferably a thermal curing process performed at a temperature which ranges from 30°C to 180°C for a period of time which ranges from 1 min to 48h.

In another preferred embodiment, the method of the invention, optionally further comprises in step a) and/or in an additional step between step c) and d), the addition of at least one additive, at least an adhesion molecule, at least a cross-linker agent and/or any combinations thereof, according to as disclosed thorough the present disclosure.

In another preferred embodiment, the excess of crosslinkers and/or surface coating molecules are washed away and the scaffolds can be used right away or are frozen and lyophilized, again for storage.

In another preferred embodiment, the sterilization of step e) is performed by a physical or chemical sterilization, preferably a physical sterilization process, which is selected from the list consisting of: hot steam, dry heat, tyndallisation, and ionizing or non-ionizing radiation, preferably hot steam. In another more preferred embodiment, the sterilization process is performed by hot steam in an autoclave for at least 20 min at a temperature for at least 121 °C at 105 kPa (1.05 bar).

In a further aspect, the present invention relates to a scaffold obtained by the method disclosed above.

Additionally, a particularly innovative fact is that the scaffold of the present invention may act as a carrier for a large variety of food supplements such as, for example, fiber, vitamins, amino acids, or likes, and it is suitable for sterilization processes without losing the tissue engineering scaffolding properties.

Thus, in another aspect, the present invention relates to the use *in vitro* of the scaffold of the present invention as a carrier.

Thus, in another aspect, the present invention refers to the use of the scaffold of the present invention for the *in vitro* production of tissues with high nutritive content and/or culture meat products.

In a further aspect, the present invention refers to a method for obtaining a tissue with high nutritive content and/or culture meat, wherein the method comprises culturing a plurality of living non-human adherent cells with a plurality of the scaffolds of the invention, in the presence of a suitable nutrient medium, wherein the cultivation takes place in a suitable bioreactor so that the initial cell population can expand and grow to form a volume of tissue and/or cultured meat. The resulting cultures may be grown to a desired level and used directly to form a tissue and/or cultured meat (e.g. by the fusion of the scaffolds of the invention), without the necessity to separate or otherwise remove the scaffolds.

In a preferred embodiment, the suitable cells for obtaining the tissue and/or the cultured meat has been disclosed above. Cells may generally be cultured with any of the edible and sterilizable scaffolds described herein in a suspension, including in a bioreactor. For example, cells may be seeded into the media along with the edible and sterilizable scaffolds and allowed to contact, adhere to, and grow on the appropriate edible and sterilizable scaffold. In some variations, culturing comprises culturing a plurality of muscle cells on edible, sterilizable and animal-product-free scaffolds wherein the edible, sterilizable and animal-product-free scaffolds comprise a flavoring, a flavor enhancer, a colorant, a color enhancer, and a nutritional enhancer.

In some variations, the scaffolds of the invention with cultured cells may be formed directly into the cultured meat product after incubation in the bioreactor for an appropriate time to allow cells to attach and proliferate (e.g., 12 hours, 24 hrs, 2 days, 3 days, 4 days, 5 days, 6 days, 7 days, 8 days, 9 days, 10 days, 11 days, 12 days, etc.). Once the cells have divided and proliferate sufficiently on scaffolds, forming a 'colonized scaffolds' in which the scaffolds are at least partially covered by (and/or filled with) cells, and the colonized scaffolds may be positioned and allowed to fuse to form the cultured meat product. In some variations a colonized scaffold is covered (e.g., greater than 50% covered, greater than 60% covered, greater than 70% covered, greater than 80% covered, greater than 90% covered, covered to confluency) with the cells.

As described previously, the cells used may be one or more types, including in particular muscle cells. Scaffolds covered to the appropriate degree with cells (e.g., >5%, >10%, >20%, >30%, >40%,>50%, >60%, >70%, >80%, >90%, etc.) may be referred to as colonized scaffolds. The colonized scaffolds may then be used to form an aggregate from which the cultured meat is formed. For example, an aggregate of the colonized scaffolds may be formed by placing colonized scaffolds immediately adjacent each other. For example, the colonized scaffolds may be placed in contact with each other so that the cells on adjacent (immediately adjacent) colonized scaffolds may contact each other and fuse to form the volume of tissue with high nutritive content and/or cultured meat.

Once the engineered meat is formed, it must be kept sterile (free from bacterial or other contamination) without the use of antibiotics, drugs, or the like, as such may impact the final meat product below. For example, the volume of tissue with high nutritive content and/or the culture meat may be frozen after it is formed.

In a further aspect, the present invention discloses the cultured meat products obtained by the method disclosed above.

In another aspect of the present invention, it relates to a tissue engineered cultured meat comprises a plurality of the scaffold according to the present invention and a plurality of living non-human cells, preferably muscle cells, and optionally, further comprises a plurality of satellite cells, stromal cells, myoblast cells, fibroblasts cells, endothelial cells, adipose cells, hepatocytes, cardiomyocytes, or any combinations thereof.

In general, these cultured meat products may include a comestible body having a volume formed of a plurality of colonized scaffolds, wherein each colonized scaffold includes an edible and sterilizable scaffold according to the present invention, further wherein the colonized scaffolds are at least partially fused to each other. As mentioned, these edible scaffolds may comprise an additive, a biocompatible molecule, a cross-linker agent and any combinations thereof. In a preferred embodiment, the cultured meat further comprising at least one additive selected from the group consisting of a flavoring, a flavor enhancer, a colorant, a color enhancer, a nutritional enhancer or any combinations thereof.

In some embodiments, the cultured meat products are fresh. In other embodiments, the cultured meat products are preserved. In further embodiments, the meat is preserved by, for example, cooking, drying, smoking, canning, pickling, salt-curing, or freezing.

In some embodiments, the cultured meat products are substantially-free of pathogenic microorganisms. In further embodiments, controlled and substantially sterile methods of cell preparation, cell culture, scaffolds preparation, and cultured meat preparation result in a product substantially-free of pathogenic microorganisms. In further embodiments, an additional advantage of such a product is increased utility and safety.

Thus, even the large volumes of cultured meat formed by the methods described herein may or may not have blood vessels. Further, the cultured meats described herein may have or may lack any nerve components (e.g., axons, dendrites, nerve cell bodies), as they may be grown with or without such components.

The cultured meat products disclosed herein are edible and intended for consumption by human beings, non-human animals, or both. In some embodiments, the cultured meat products are human food products. In other embodiments, the cultured meat products are animal feed such as feed for livestock, feed for aquaculture, or feed for domestic pets. Therefore, in light of the disclosure provided herein, those of skill in the art will recognize that non-human cells from a plethora of sources are suitable for use in production of such products and with the methods disclosed herein. In various embodiments, the scaffolds and the cultured meat products comprise non-human cells derived from, by way of non-limiting examples, mammals, birds, reptiles, fish, crustaceans, cephalopods, insects, non-arthropod invertebrates, and combinations thereof.

In this text, the term "comprises" and its derivations (such as "comprising", etc.) should not be understood in an excluding sense, that is, these terms should not be interpreted as excluding the possibility that what is described and defined may include further elements, steps, etc.

On the other hand, the invention is obviously not limited to the specific embodiment(s) described herein, but also encompasses any variations that may be considered by any person skilled in the art (for example, as regards the choice of materials, dimensions, components, configuration, etc.), within the general scope of the invention as defined in the claims.

### BRIEF DESCRIPTION OF THE DRAWINGS

To complete the description and in order to provide for a better understanding of the invention, a set of drawings is provided. Said drawings form an integral part of the description and illustrate an embodiment of the invention, which should not be interpreted as restricting the scope of the invention, but just as an example of how the invention can be carried out. The drawings comprise the following figures:
**Figure 1****.** Cylindrical scaffolds of different lengths and diameters formed by molding. Scaffolds a), b) and c) corresponding to Example 1, 2 and 3, respectively.
**Figure 2****.** Schematic description of extrusion into a frozen surface. Inlet shows the final pellets that can be produced by this method.
**Figure 3****.** Phase diagram of water indicating the triple point; wherein the lyophilization process will occur below of this triple point.
**Figure 4****.** Scanning electron microscopy (SEM) images of porous scaffolds of Example 1 (**4a**), Example 2 (**4b**) and Example 3 (**4c**) before cell seeding. Tissue proliferation over scaffolds of Example 1 (**4d**), Example 2 (**4e**) and Example 3 (**4f**).
**Figure 5****.** Mercury (Hg) porosimetry studies before (**A**) and after (**B**) sterilization process of the scaffold disclosed in Example 1. Data of total porosity and total surface area.
**Figure 6****.** Mercury (Hg) porosimetry studies before (**A**) and after (**B**) the sterilization process of the scaffold disclosed in Example 2. Data of total porosity and total surface area.
**Figure 7****.** Mercury (Hg) porosimetry studies before (**A**) and after (**B**) sterilization process of the scaffold disclosed in Example 3. Data of total porosity and total surface area.
**Figure 8****.** FTIR (Fourier Transform-Infrared Spectroscopy) spectra of samples before (top line) and after (bottom line) sterilization procedure corresponding to scaffolds from Example 1 (**8A**), 2 (**8B**) and 3 (**8C**), respectively.
**Figure 9.** Figure shows glucose consumption (expressed al g/L) at different assay times (expressed as days) by the mammal cells sown in the scaffold obtained in the Examples 1 to 3 of the invention (Example 1, Example 2 and Example 3, in the legend)

in comparison to the initial glucose (initial, in the legend) and the culture controls in the absence of the scaffolds of the invention (control, in the legend).

### EXAMPLES

Following are examples of the invention by means of assays carried out by the inventors, which evidence the effectiveness of the product of the invention. The following examples serve to illustrate the invention and must not be considered to limit the scope thereof.

### Example 1. Synthesis of the scaffold of the present invention comprising chitosan as biocompatible polymer.

In a typical production method of the edible and sterilizable macroporous 3D scaffold that will be equally valid for other biocompatible polymers and mixtures thereof, commercial MEM Eagle (Minimum Essential Medium Eagle) w/Earle's BSS w/Non-Essential aminoacids & L-glutamine, w/o Calcium in powder (ranging among 0-600 mg, to achieve 0-200% related to the chitosan weight, more preferably 75 mg, 10% related to the chitosan weight), was dissolved in acetic acid (0.1 M, 50 mL) and chitosan (750 mg, 1.5%) was slowly added while stirring. The mixture was stirred until homogeneous solutions were obtained (65°C, 2 hours) and the solution was poured into molds to give the desired shape and dimensions. All the samples were frozen at -20°C for 24 hours and lyophilized at -40°C during 72 hours, at a pressure of 0.263 mbar.

This is a method for manufacturing scaffolds with controlled shapes, which will be determined by the receptacle wherein the process is carried out.

The scaffolds were removed from the molds and put inside the oven at 135°C for 1 hour without ventilation (the curing starts by pre-heating the oven at 45°C, as soon as the oven reaches 135°C time starts; after 1 hour the scaffolds are left to reach room temperature inside the oven, it takes around 1h 30 minutes. At this stage, the scaffolds were washed with miliQ water (3x100 mL for 15 minutes), 0.1 M NaHCO₃ (3x100 mL for 15 minutes) and water (3x100 mL for 15 minutes).

The results show that the scaffold of the invention which comprises MEM powder (0%) loses its consistency when it is immersed in water. Therefore, it seems that the cross-linking temperature and time was not enough to obtain a scaffold with the desired properties regarding mechanical strength and appearance. However, the results show that the remaining scaffolds comprising different MEM concentrations (1%, 8%, 16%, 33%, 133%, 200% more preferably 8%) display good consistency and do not lose their shape. These data state that the components of the MEM are also acting as crosslinkers giving consistency to the otherwise breakable scaffolds.

Later, the scaffolds with an 8% MEM concentration were sterilized in an autoclave at 121°C for 20 minutes at 1.05 bar and tested. The scaffolds are stable and some degree of cross-linking has happened because the scaffolds do not swell in excess, this is the cross-linking reduces the swelling.

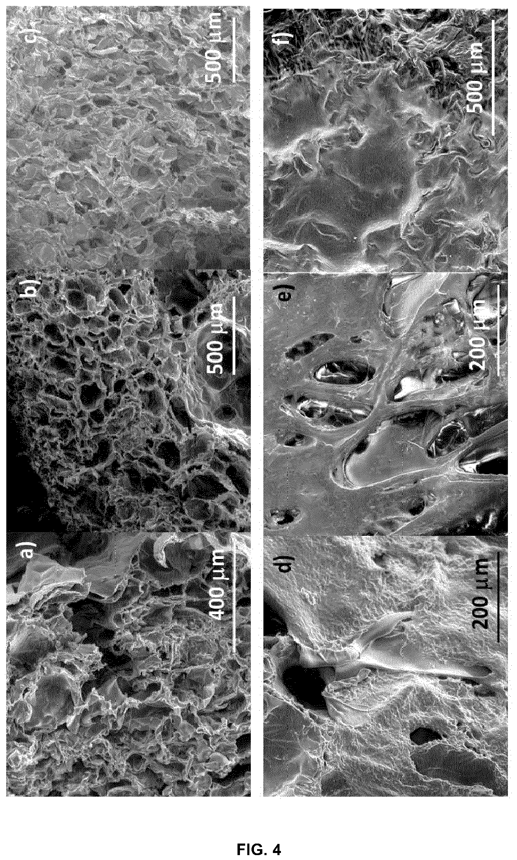

The macroporosity of these scaffolds of the invention having a percentage by weight/volume of the chitosan polymer (1.5%), could be controlled and shows a high pore volume and total porosity of 90% giving rise to foam-like samples. The optimized scaffold exhibits a high volume and size of inter-connected macroporosity in the range 1-500 µm as can be observed by Digital Imaging (**Fig**. **1a****)** and SEM (**Fig. 4a**).

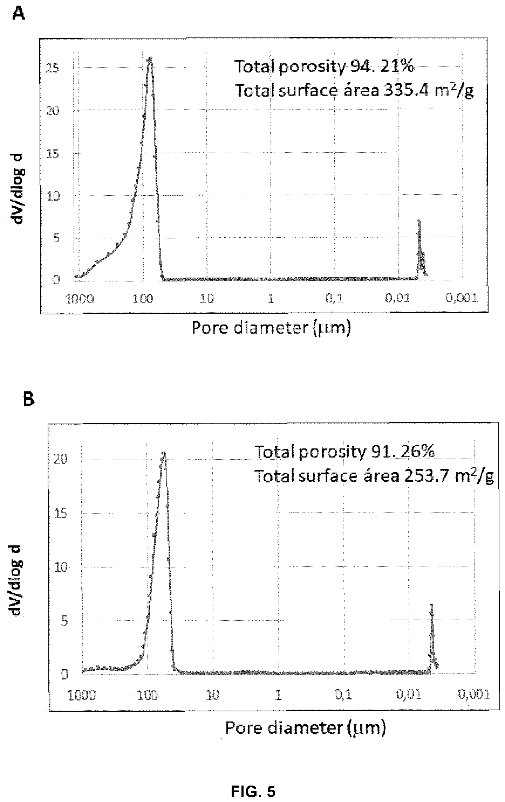

In **Fig. 5** it is show the Hg (mercury) intrusion porosity analysis of the scaffold obtained in the instant example. This technique is used to analyze the total connected porosity, volume of pores, pore size distribution, and surface area of solid and powder materials. The instrument, known as a porosimeter, employs a pressurized chamber to force mercury to intrude into the voids in a porous substrate. As pressure is applied, mercury fills the larger pores first. As pressure increases, the filling proceeds to smaller and smaller pores. Both the inter-particle pores (between the individual particles) and the intra-particle pores (within the particle itself) can be characterized using this technique. The volume of mercury intruded into the sample is monitored by a capacitance change in a metal clad capillary analytical cell called a penetrometer. The sample is held in a section of the penetrometer cell, which is available in a variety of volumes to accommodate powder or intact solid pieces. Sample size is limited to dimensions of approximately 2.5 cm long by 1.5 cm wide. This powerful technique was used to analyze the change on the porous nature of the scaffold before (**Fig. 5A**) and after (**Fig. 5B**) sterilizing the pieces. As can be seen in **Fig. 5****,** results show a slight decrease of total pore volume and in the total surface area due to shrinking from the sterilization step. Still, the values after sterilization are more than highly suitable for the application showing the right pore volume range and high surface area available for cell colonization.

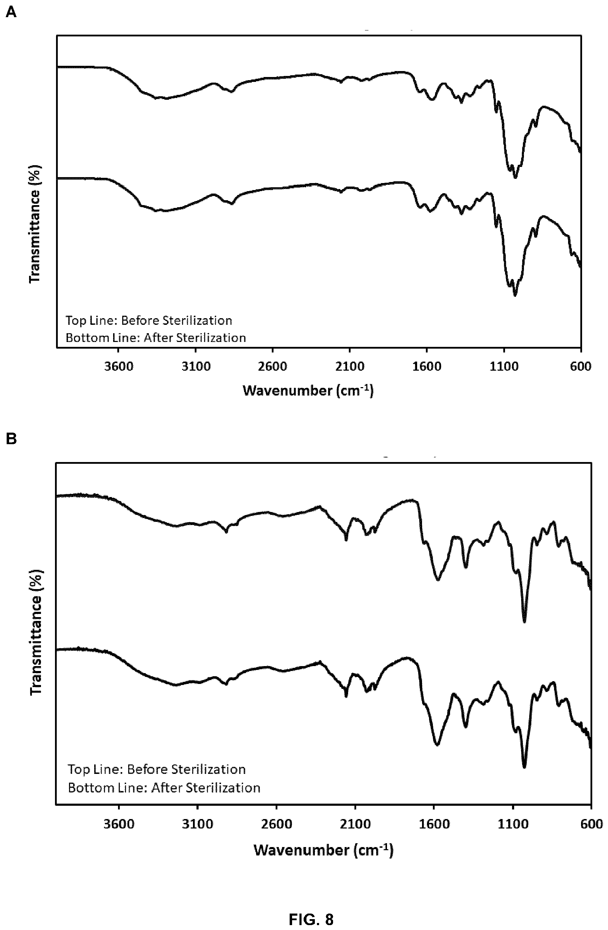

In order to prove that non-chemical modifications have occur during hot steam sterilization procedure that could compromise scaffolds suitability for cell culture, scaffolds before and after sterilization have been analyzed by FTIR (Fourier Transform-Infrared Spectroscopy). FTIR is an analytical technique used to identify organic (and in some cases inorganic) materials. This technique measures the absorption of infrared radiation by the sample material versus wavelength. The infrared absorption bands identify molecular components and structures. As it can be seen in **Fig. 8A** there are no changes on the band spectrum shape and position before and after the sterilization process proving the suitability of the chemical identity of the scaffolds.

### Example 2. Synthesis of the scaffold of the invention comprising alginate as biocompatible polymer and coated with poly-ε-Lysine.

Alginic acid sodium salt (Sigma- Aldrich) at a concentration of 2.0 g was slowly added under vigorous stirring to water (200 mL). The mixture was stirred at 50 °C for 3 hours until a homogeneous and transparent and clear solution was obtained. The solution was left to reach room temperature.

Later, the solution was poured into molds to give the desired shape and dimensions. All the samples were frozen at -20°C for 24 hours and lyophilized at -40°C for 72 hours at a pressure of 0.263 mbar.

The lyophilized scaffolds obtained were removed from the molds and immersed in a 2% aqueous solution of calcium chloride (4 g, 200 mL) (any other aqueous solution can be used, such as, calcium sulfate, calcium carbonate, calcium gluconate, calcium citrate) in miliQ water (200 mL) for 15 minutes to provoke the crosslinking of the polymer. In this solution, the alginate scaffolds gelled immediately and washed extensively with miliQ water to remove the unbound calcium ions. The scaffolds were then immersed in a 0.2% poly-epsilon-lysine solution (1.0 g in 500 mL) for 16 hours, and then washed extensively with miliQ water. The scaffolds were frozen again at - 20°C for 24 hours and lyophilized at -40°C for 72 hours and a pressure of 0.263 mbar, and later, stored until used.

The lyophilized scaffolds were later heated in an oven at 115°C for 1 hour without ventilation (the curing starts by pre-heating the oven at 45°C, as soon as the oven reaches 115°C time starts; after 1 hour the scaffolds are left to reach room temperature inside the oven, it takes around 1h 30 minutes).

Later, the scaffolds were sterilized in an autoclave at 121°C for 20 minutes at 1.05 bar.

The optimized scaffold exhibits a high volume and size of inter-connected macroporosity in the range 1-500 µm as can be observed by Digital Imaging (**Fig. 1b**) and SEM (**Fig. 4b**).

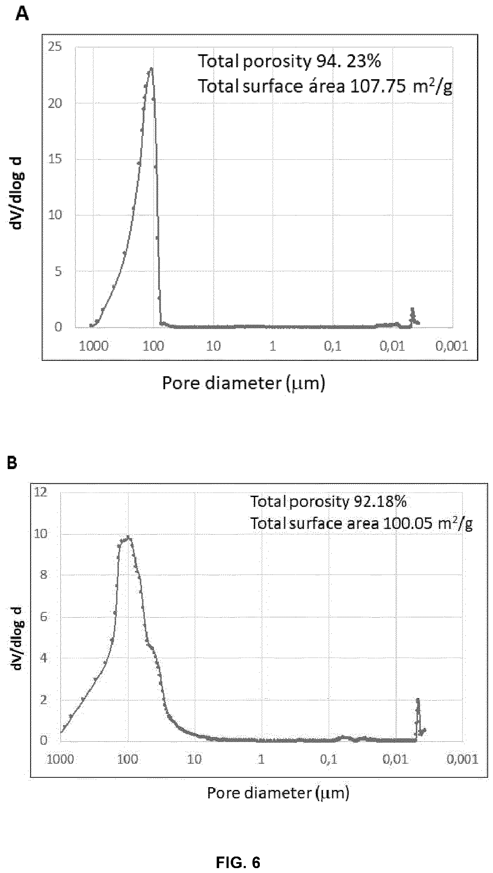

As shown in **Fig.6****,** the Hg (mercury) intrusion porosity analysis, before (**Fig. 6A**) and after (**Fig. 6B**) sterilizing the pieces, shows a slight decrease of total pore volume and in the total surface area. Still, the values after sterilization are highly suitable for the application showing the right pore volume range and high surface area available for cell colonization.

As it can be seen in **Fig. 8B** there are no changes on the band spectrum shape and position before and after the sterilization process proving the suitability of the chemical identity of the scaffolds.

### Example 3. Synthesis of the scaffold of the invention comprising chitosan as biocompatible polymer and coated with poly-ε-Lysine.

Food grade chitosan (9.0 g) was mixed with acetic acid (0.1 M, 600 mL) under gentle stirring at 40°C until chitosan was dissolved. Poly-ε-L-lysine (360 mg powder, M_{w}:1000-300000) was portion wise added to the previous solution under vigorous stirring and the mixture was warmed to 65 °C for 2 hours and 30 minutes to obtain a homogeneous and clear solution.

The previous solution was left to reach room temperature and it was poured into the molds to give the scaffolds the desired shape and size (**Fig. 1c**). The solution inside the molds was frozen at -20°C for 24 hours and lyophilized at -40°C for 72 hours and a pressure of 0.263 mbar.

The scaffolds were removed from the molds and they were jellified with ethanol absolute (2 litres) for 4 hours. The scaffolds were washed with high purity water and the scaffolds were immersed in a NaOH solution (1%) for 3 hours. The scaffolds were extensively washed with high purity water until the lixiviate reaches a neutral pH (6.5-7.5). The so formed scaffolds are frozen at -20°C for 24 hours and lyophilized (72 hours, -40°C and P = 0.263 mbar). The scaffolds were directly used without any further treatment or alternatively, it can be cured. Cured scaffolds were subjected to a heat treatment by introducing them inside an oven at 110°C for 1 hour without ventilation (the curing starts by pre-heating the oven at 45°C, as soon as the oven reaches 110°C time starts; after 1 hour the scaffolds are left to reach room temperature inside the oven, it takes around 1h 30 minutes). Later, the scaffolds were sterilized in an autoclave at 121°C for 20 minutes at 1.05 bar. The optimized scaffold exhibits a high volume and size of inter-connected macroporosity in the range 1-500 µm as can be observed by Digital Imaging **(****Fig.1c****)** and SEM (**Fig. 4c**).

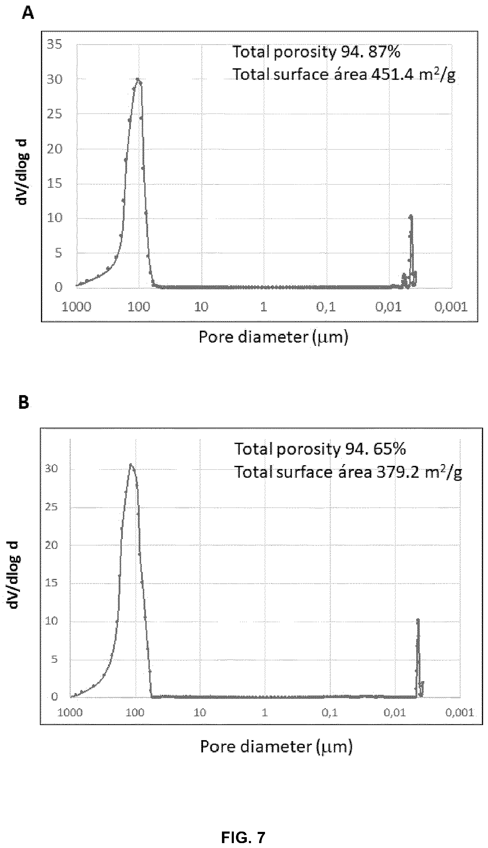

As shown in **Fig.7****,** the Hg (mercury) intrusion porosity analysis, before (**Fig. 7A**) and after (**Fig. 7B**) sterilizing the pieces, shows a slight decrease of total pore volume and in the total surface area. Still, the values after sterilization are highly suitable for the application showing the right pore volume range and high surface area available for cell colonization.

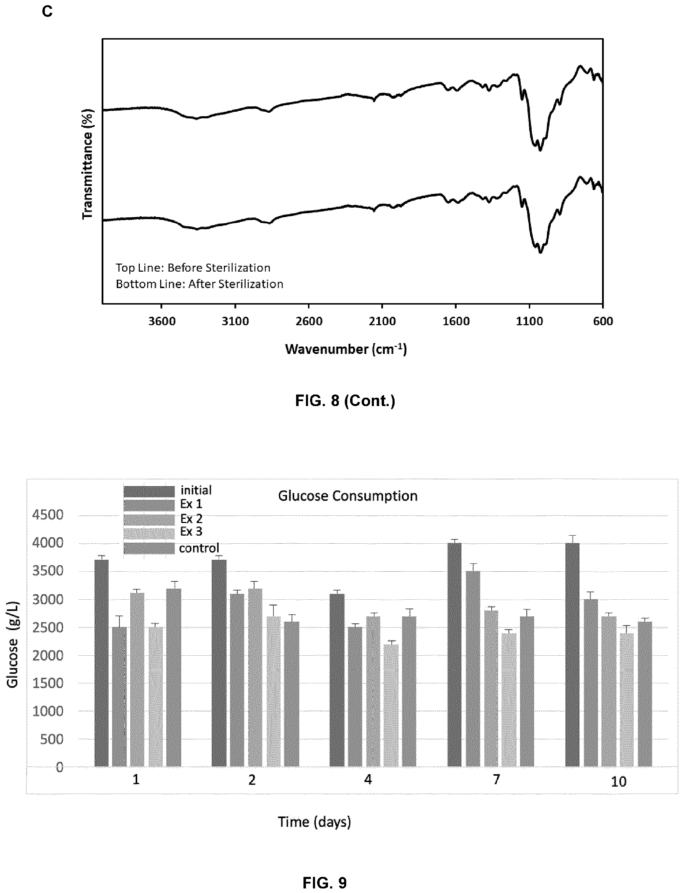

As it can be seen in **Fig. 8C** there are no changes on the band spectrum shape and position before and after the sterilization process proving the suitability of the chemical identity of the scaffolds.

### Example 4. Culture of the scaffolds of Examples 1 to 3 of the invention with non-human cells in a bioreactor.

To verify that the chitosan based-scaffolds (Examples 1 and 3) and alginate based-scaffolds (Example 2) obtained by the method of the invention are useful for obtaining cultured meat, the inventors cultured living cells along the mentioned scaffolds of the invention in a bioreactor.

To this end, the scaffolds of the Examples 1, 2 and 3 were sterilized in an autoclave at 121°C for 20 minutes, at 1.05 bar, and later sterilely immersed in a final volume of culture medium of 2 liters (Gibco^{™} OptiPRO^{™}). Next, a known cell density (13.000 cells/cm²) was inoculated in the mentioned scaffolds and kept under culture for long periods of time (10 days or more) in a 48-hour perfusion bioreactor. The inoculated cells were primary porcine-myoblast cells, of proprietary extraction obtained from muscle biopsies and extracted following the protocol described in JM Spinazzola et al., Bio Protoc. 2017 November 5; 7(21). The culture conditions for adequate cell proliferation require maintaining a temperature of 37 °C and a pH value of 7.1-7.4. Additionally, a continuous supply of gases is maintained, such that the concentration of dissolved oxygen ranges between 20% - 30%. After a period of 10 days, the scaffolds were removed from the bioreactor and dehydrated with ethanol in order to perform SEM (Scanning Electron Microscopy) visualization. SEM images (**Fig 4 d**, **e and f**), show the full tissue proliferation over the scaffold of Example 1, Example 2 and Example 3, respectively.

During the incubation period of 10 days, glucose consumption was measured with a Bioprofile Analyzer with a Glucose biosensor. Glucose biosensors are amperometric electrodes that have immobilized enzymes in their membranes. In the presence of oxygen and the substrate being measured, these enzyme membranes produce hydrogen peroxide (HzOz), which is then oxidized at a platinum anode held at constant potential. The resulting flow of electrons and current change is proportional to the sample glucose concentration. Measurements were carried out at different assay times as an indirect measurement of the proliferation and formation of tissue over the scaffold of the present invention. The data obtained are shown in **Fig. 9.** The results show the existence of glucose consumption by the primary porcine-myoblast cells sown in the matrix of the invention over time (1-10 assay days), since a decrease in glucose concentration values is observed compared to the initial glucose levels of the culture medium (approximately 4 g/L). Positive controls were performed on each assay day.

Thus, the results show that the scaffolds of the present invention are useful for cell culture and for obtaining *in vitro* tissue with high nutritive content and/or cultured meat.

## Claims

1. An edible and sterilizable by hot steam macroporous three-dimensional tissue engineering scaffold comprising a biocompatible polymer from natural o synthetic origin or any variant thereof, wherein the biocompatible polymer is preferably from natural origin.

2. The scaffold according to claim 1 wherein, the natural polymer or any variant thereof is selected from the list consisting of: dextran, alginate, chitosan, starch, heparin, heparin sulfate, pullulan, cellulose, hemicellulose, glucomannan, agar, chondroitin sulfate, gelatin, chitin, polynucleotides, a polysaccharide, a glycosaminoglycan, natural polyesters, or any combinations thereof, preferably, the natural polymer or any variant thereof is selected from chitosan and/or alginate.

3. The scaffold according to claim 1 wherein the synthetic polymer or any variant thereof is selected from the list consisting of: polylactic acid, polyglycolic acid, poly(lactic-co-glycolic) acid, polycaprolactone, polyhydroxyalkanoates, bioesters, or any combinations thereof.

4. The scaffold according to any of claims 1 to 3 wherein, the macropores are hierarchical and interconnected and have an average pore size which ranges from 1 µm to 10000 µm, preferably from 50 to 1000 µm, more preferably from 100 µm to 1000 µm, or even more preferably from 100 µm to 500 µm.

5. The scaffold according to any of claims 1 to 4 wherein is sterilized alternatively by a physical or a chemical process, preferably a physical sterilization process.

6. The scaffold according to claim 5 wherein, the physical sterilization is selected from the list consisting of: dry heat, tyndallisation, and ionizing or non-ionizing radiation.

7. The scaffold according to any of claims 1 to 6 wherein, further comprises non-human living cells which are homogeneously distributed though the scaffold.

8. The scaffold according to claim 7 wherein, the non-human living cells are adherent cells, preferably selected from the list consisting of: muscle cells, endothelial cells, adipose cells, hepatocytes, cardiomyocytes or likes.

9. The scaffold according to any of claims 1 to 8 wherein, further comprises at least one additive, at least a bioactive molecule, at least a cross-linker agent and/or any combinations thereof.

10. The scaffold according to claim 9 wherein the additive is selected from the list consisting of: flavoring, a flavor enhancer, a colorant, a color enhancer, salts, acidity regulators, thickeners, emulsifiers, stabilizer, a nutritional enhancer, probiotics, prebiotics, saponins, antioxidants, essential fatty acids, minerals, and any combinations thereof.

11. The scaffold according to claim 9 wherein the bioactive molecule is selected from the list consisting of: inmuglobulin-superfamiliy proteins, cadherins, selectins or integrins; fibronectins, poly-L-ornithine, collagen, vitronectins, lectin, poly-ornithine, poly-L-lysine, poly-D-lysine, cyclic peptides, RGD-containing peptides, RGDS-containing peptides or any combinations thereof, preferably the bioactive molecule is poly-L-lysine, more preferably is poly-ε-lysine.

12. The scaffold according to claim 9 wherein, the cross-linker agent is selected from the list consisting of amine groups, hydroxyl groups, carbonyl, groups, aldehyde groups, carboxylate group, carbonate group, carboxyl groups, carboxamide groups, imine groups, imide groups, thiol groups, inorganic ions and any combinations thereof

13. The scaffold according to any of claims 1 to 12 wherein, is in the form of hydrogel.

14. Use of the scaffold according to any of claims 1 to 13 in the *in vitro* production of a tissue and/or a cultured meat.

15. Use according to claim 14 wherein the cultured meat comprises a plurality of adherent cells, preferably muscle cells and optionally, further comprises a plurality of satellite cells, stromal cells, fibroblasts cells, myoblast cells, endothelial cells, adipose cells, hepatocytes, cardiomyocytes or any combinations, or any combinations thereof.

16. Use according to claim 15 wherein the cells belong to an animal source selected from the list consisting of: mammals preferably porcine, bovine, ovine, horse, dog, cat; avian; reptile; fish; amphibians; cephalopods, crustaceans or any combinations thereof.

17. Use according to any of claims 14 to 16 wherein the scaffold does not have to be removed from the final ready-to-eat cultured meat.

18. Use *in vitro* of the scaffold according to any of claims 1 to 13 as carrier.

19. A method for obtaining a culture meat, wherein the method comprises culturing a plurality of living non-human cells, preferably muscle cells with a plurality of the scaffolds according to any of claims 1 to 13, in the presence of a suitable nutrient medium.

20. A method for obtaining a culture meat according to claim 19, wherein the cultivation takes place in a bioreactor.

21. A cultured meat comprises a plurality of the scaffold according to any of claims 1 to 13 and a plurality of living non-human cells, preferably muscle cells, and optionally, further comprises a plurality of satellite cells, myoblast cells, myofibroblast cells, fibroblasts cells, endothelial cells, adipose cells, hepatocytes, cardiomyocytes or any combinations thereof.

22. A cultured meat according to claim 21 further comprising at least one additive selected from the group consisting of a flavoring, a flavor enhancer, a colorant, a color enhancer, a nutritional enhancer or any combinations thereof.

23. A cultured meat according to any of claims 21 to 22 wherein the cultured meat comprises a plurality of the colonized scaffolds immediately adjacent each other.

24. A cultured meat according to any of claims 21 to 23 wherein the cultured meat is substantially-free of pathogenic microorganisms.
